# EUROPEAN PATENT SPECIFICATION

(11) **EP 2 923 631 B1**
(45) Date of publication and mention of the grant of the patent: **30.11.2016**
(21) Application number: 15159135.1
(22) Date of filing: 16.03.2015
(51) Int. Cl.: A61B 1/005, A61B 1/12

(54) **ENDOSCOPE INSERTION PORTION AND ENDOSCOPE**
ENDOSKOPEINFÜHRABSCHNITT UND ENDOSKOP
PARTIE D'INSERTION D'ENDOSCOPE ET ENDOSCOPE

(30) Priority: 26.03.2014 JP 2014063996
(43) Date of publication of application: 30.09.2015
(73) Proprietor: FUJIFILM Corporation, Tokyo 106-8620 (JP)
(72) Inventor: Ikeda, Toshiyuki, Kanagawa, 258-8538 (JP); Torii, Yuichi, Kanagawa, 258-8538 (JP); Iyama, Shozo, Kanagawa, 258-8538 (JP)
(74) Representative: Klunker . Schmitt-Nilson . Hirsch

(56) References cited:
- JP-A- H11 253 393
- US-A1- 2002 040 181
- US-A1- 2013 253 268

## Description

### BACKGROUND OF THE INVENTION

### 1. Field of the Invention

The present invention relates to an endoscope insertion portion and an endoscope equipped with a merging member that merges a liquid supply tube with a gas supply tube and communicates with a gas/liquid supply tube.

### 2. Description of the Related Art

US 2002/0040181 A1 represents the closest prior art and teaches that in a tube connecting structure of an endoscope, a fluid supply tube is connected to a gas supply tube and a liquid supply tube via a tube joint. The tube joint is arranged at the inside of an insertion part of the endoscope, and is constructed of a main tube and a branch tube.

Endoscopes are equipped with an endoscope insertion portion to be inserted into the inside of a subject's body, and a proximal operating portion that is provided continuously with a proximal end of the endoscope insertion portion. The endoscope insertion portion includes a distal end portion, a bendable portion, and a flexible portion sequentially from the distal end of the insertion portion. The bendable portion has a structure in which a plurality of bendable pieces are coupled together, and is operated in a bendable manner by a bendable operation wire provided inside bendable pieces being pushed and pulled, and thereby the orientation of a distal end portion thereof is changed.

The distal end portion of the endoscope insertion portion is equipped with an observation window, an illumination window, and a fluid jet nozzle. An imaging element for imaging the inside of a subject's body is provided in the back of the observation window. The illumination window allows illumination light to be radiated therethrough. The fluid jet nozzle has a jet port at a distal end thereof, and selectively jets, for example, liquids, such as cleaning water, or gases, such as air or carbon dioxide gas therefrom. A liquid jetted from the fluid jet nozzle flushes away dirt on the observation window, and a gas blows away droplets remaining on the surface of the observation window.

The fluid jet nozzle is connected to a gas/liquid supply tube disposed inside the endoscope insertion portion. This gas/liquid supply tube communicates with a liquid supply tube and a gas supply tube, respectively, through which that a liquid and a gas are supplied, via a merging member. The merging member has branch pipe portions that branch on a proximal end side and are connected to the liquid supply tube and the gas supply tube, and a merging pipe portion that is provided continuously with the branch pipe portions and is connected to the gas/liquid supply tube.

In an endoscope described in JP1999-253393A (JP-H11-253393A), a merging member is disposed within a proximal operating portion. Additionally, in the andoscope described in JP1999-253393A (JP-H11-253393A), the merging member is disposed inside an enlarged portion provided between an endoscope insertion portion and the proximal operating portion, a gas/liquid supply tube is disposed within the endoscope insertion portion, and a liquid supply tube and a gas supply tube are disposed within the proximal operating portion.

When fluid jetting is performed from a fluid jet nozzle onto an observation window, a gas is supplied and a liquid is pushed out from the inside of the gas/liquid supply tube after the liquid is supplied to the gas/liquid supply tube. However, in a case where the gas/liquid supply tube is too long, when switching is done from a liquid supply operation to a gas supply operation, the gas is jetted from the fluid jet nozzle after the liquid that has remained within the gas/liquid supply tube is pushed out. Therefore, a surgeon feels the time lag for the gas supply operation. Additionally, there is a concern that droplets that remain in the gas/liquid supply tube during the gas supply operation may be jetted from the fluid jet nozzle and may adhere to the observation window together with the gas. In the endoscopes described in JP1999-253393A (JP-H11-253393A) and JP2001-70230A, the merging member is located closer to a proximal end side than the endoscope insertion portion, the gas/liquid supply tube is long, and the liquid may remain therein. Therefore, it is required to make the gas/liquid supply tube as short as possible. Thus, in endoscopes described in JP2002-102154A, JP1988-031631A (JP-S63-031631A), and JP1996-010216A (JP-H08-010216A), a merging member is disposed within an endoscope insertion portion.

### SUMMARY OF THE INVENTION

However, a plurality of built-in elements, such as a signal cable connected to a circuit board of the imaging element, a light guide that guides illumination light to the illumination window, the bendable operation wire that bends the bendable portion, and the like, are inserted through the inside of the endoscope insertion portion. Particularly, since the inside of the bendable piece located on the most proximal end side of the bendable portion is provided with a fixing portion that fixes the guide member inserted through the bendable operation wire, the packing ratio within the bendable piece is high. When the merging member is arranged within the endoscope insertion portion as in the endoscopes described in JP2002-102154A, JP1988-031631A (JP-S63-031631A), and JP1996-010216A (JP-H08-010216A), if the liquid supply tube and the gas supply tube or the branch pipe portion of the merging member is arranged within the bendable portion, the packing ratio in this case may become higher than that in a case where the gas/liquid supply tube is arranged therein. As a result, the bending of the bendable portion may be hindered.

The invention has been made in view of the above problems, and an object thereof is to provide an endoscope insertion portion and an endoscope in which a merging member does not hinder the operation of a bendable portion.

An endoscope insertion portion of the invention is defined in the independent claims 1 and 3. It has a distal end portion, a bendable portion, and a flexible portion sequentially from the distal end of the insertion portion. The endoscope insertion portion includes a fluid jet nozzle, a gas supply tube and a liquid supply tube, a plurality of bendable pieces, a coupling ring, a merging member, and a gas/liquid supply tube. The fluid jet nozzle is provided at the distal end portion to jet a gas and a liquid therefrom. The gas supply tube and the liquid supply tube supply the gas and the liquid from a gas/liquid supply device provided outside the endoscope insertion portion. The plurality of bendable pieces are provided at the bendable portion and coupled together in series. The coupling ring is provided on a most distal end side of the flexible portion and is coupled to a proximal end piece which is one of the bendable pieces provided on a most proximal end side among the bendable pieces. The merging member has branch pipe portions and a merging pipe portion. Branch pipe portions branch on the proximal end side and communicate with the gas supply tube and the liquid supply tube, respectively. The merging pipe portion is provided continuously with the branch pipe portions. The gas/liquid supply tube allows the fluid jet nozzle and the merging pipe portion to communicate with each other and has a length such that the merging pipe portion stays in the coupling ring when the merging member is pulled in to the distal end portion side by bending the bendable portion. In addition, preferably, a portion of the merging pipe portion is located in the flexible portion in a state where the bendable portion is linear.

In one alternative the endoscope insertion portion further includes built-in elements to be inserted through the bendable portion and the flexible portion together with the merging member. The proximal end piece may have therein a space where the merging pipe portion or the gas/liquid supply tube fitted to an outer peripheral surface of the merging pipe portion enters, and the entry of the branch pipe portions into the proximal end piece may be regulated in a state where the built-in elements have abutted against the branch pipe portions.

In the other alternative, the endoscope insertion portion includes a bendable operation wire, a guide member, and a fixing portion. The bendable operation wire may be provided for bendingly operating the bendable portion. The guide member may guide the bendable operation wire. The fixing portion may be provided inside the proximal end piece and fixes the guide member therein. The entry of the branch pipe portion into the proximal end piece may be regulated in a state where the fixing portion has abutted against the branch pipe portion.

Preferably, the fixing portion is a bent portion formed by bending a part of a cylinder portion of the proximal end piece inward. The fixing portion may have a protruding portion that is formed integrally with the bent portion and may be made to protrude toward the merging member. The fixing portion may regulate the entry of the branch pipe portion into the proximal end piece in a state where the protruding portion has abutted against the branch pipe portion.

Preferably, the endoscope insertion portion further includes a biasing member that is provided between the guide member and the merging member. The biasing member may bias the merging member to a proximal end side of the flexible portion to regulate the entry of the branch pipe portion into the proximal end piece. Additionally, preferably, the gas/liquid supply tube, the liquid supply tube, the gas supply tube, and the merging member are integrally formed.

An endoscope of the invention includes the endoscope insertion portion, a hand operating portion, and a bending operation member. The hand operating portion is provided continuously with the endoscope insertion portion. The bendable operation member performs the bending of the bendable portion.

According to the invention, since the merging pipe portion of the merging member and the fluid jet nozzle communicate with each other via the gas/liquid supply tube, and the gas/liquid supply tube has a length such that the merging pipe portion stays in the coupling ring when the merging member is pulled in to the distal end portion side by the bending of a bendable portion, the tube length of the gas/liquid supply tube can be sufficiently shortened. Moreover, as the merging pipe portion stays in the coupling ring, the entry of the branch pipe portions into the bendable portion having a high packing ratio can be prevented, and the endoscope insertion portion and the endoscope that do not hinder the operation of the bendable portion can be provided.

### BRIEF DESCRIPTION OF THE DRAWINGS

Fig. 1 is a perspective view illustrating an embodiment of an endoscope system.
Fig. 2 is a conduit map illustrating conduits inside an electronic endoscope illustrated in Fig. 1.
Fig. 3 is a cross-sectional view of essential portions of a distal end portion and a bendable portion.
Fig. 4 is a perspective view illustrating the distal end portion of the electronic endoscope.
Fig. 5 is a cross-sectional view of essential portions of a bendable portion and a flexible portion.
Fig. 6 is an exploded perspective view illustrating the configuration of a joining portion between the bendable portion and the flexible portion.
Fig. 7A is an explanatory view illustrating an endoscope insertion portion in a state where the bendable portion is linear.
Fig. 7B is an explanatory view illustrating the endoscope insertion portion in which the bendable portion is brought into a bent state and into which a gas/liquid supply tube and a merging member are pulled.
Fig. 8A is a cross-sectional view orthogonal to a cylinder center direction at a position of a proximal end piece of the bendable portion.
Fig. 8B is a cross-sectional view orthogonal to the cylinder center direction at a position of a coupling ring of the flexible portion.
Fig. 9 is a back side perspective view illustrating a proximal end piece of a bendable portion of a second embodiment.
Fig. 10 is a cross-sectional view orthogonal to a cylinder center direction at a position of the proximal end piece of the second embodiment.
Fig. 11 is a perspective view illustrating a configuration in which a spring that biases a merging member of a third embodiment is provided.

### DESCRIPTION OF THE PREFERRED EMBODIMENTS

### <First Embodiment>

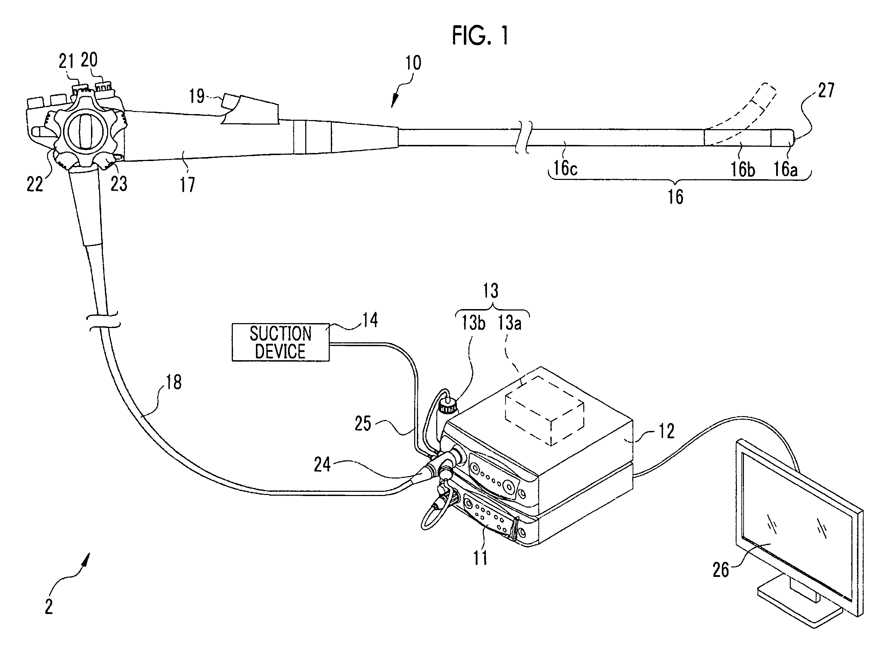

As illustrated in Fig. 1, an endoscope system 2 is equipped with an electronic endoscope 10, a processor device 11, a light source device 12, a gas/liquid supply device 13, and a suction device 14. The gas/liquid supply device 13 has a well-known gas supply device (pump or the like) 13a that is built in the light source device 12 and performs supply of gas, and a liquid tank 13b that is provided outside the light source device 12 and stores a liquid. The electronic endoscope 10 has a flexible insertion portion 16 for an endoscope (hereinafter referred to an "insertion section") to be inserted into the inside of the body, a hand operating portion 17 provided continuously with a proximal end portion of the insertion portion 16, and a universal cord 18 connected to the processor device 11 and the light source device 12. The liquid to be supplied by the gas/liquid supply device 13 is cleaning water, and the gas to be supplied is air or carbon dioxide gas.

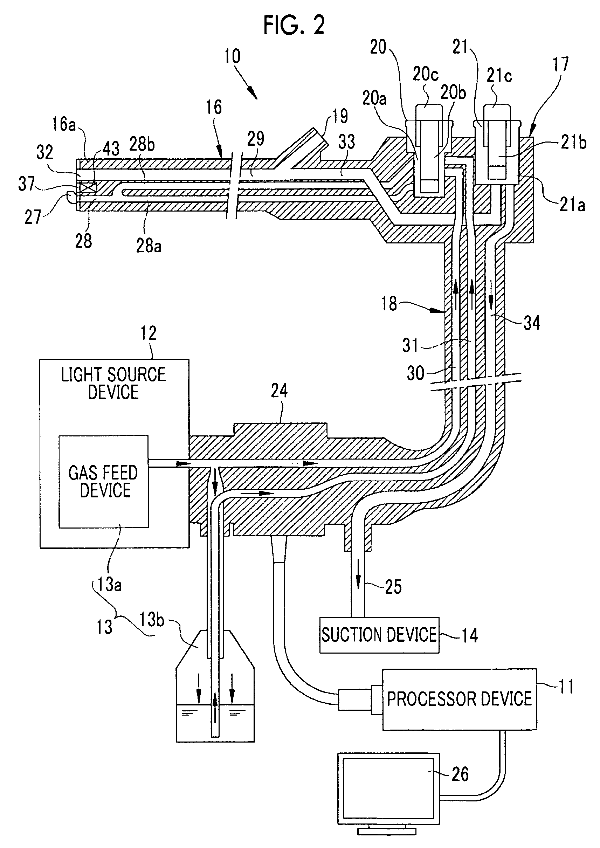

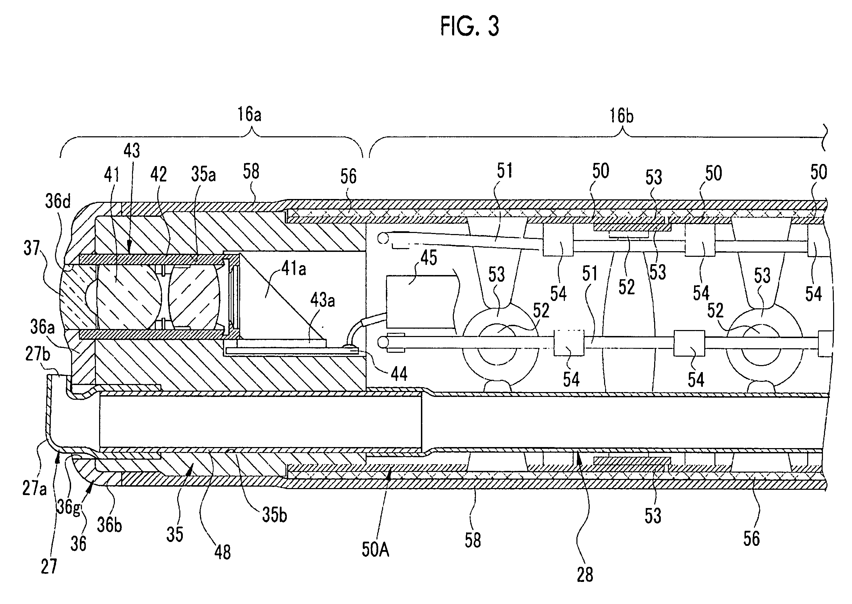

The insertion portion 16 has a distal end portion 16a, a bendable portion 16b, and a flexible portion 16c sequentially from the distal end from the insertion portion 16. A camera unit 43 (refer to Figs. 2 and 3) for imaging the inside of a subject's body is built in the distal end portion 16a. The bendable portion 16b is provided continuously with a proximal end of the distal end portion 16a, and is configured in a bendable manner. The flexible portion 16c is provided continuously with a proximal end of the bendable portion 16b, and has flexibility.

The hand operating portion 17 is equipped with a treatment tool inlet 19, a gas/liquid supply button 20, a suction button 21, and bendable operation knobs 22 and 23 serving as bendable operation members. A connector 24 is attached to the other end of the universal cord 18. The connector 24 is a complex type connector, and the processor device 11, the light source device 12, and the gas/liquid supply device 13 are connected to the connector, respectively. The suction device 14 is connected to the connector 24 via a coupling tube 25.

The processor device 11 is electrically connected to the light source device 12, and generally controls the operation of the endoscope system 2. The processor device 11 supplies electric power to the electronic endoscope 10 via a transmission cable inserted into the universal cord 18 and the insertion portion 16, and controls the driving of the camera unit 43. Additionally, the processor device 11 acquires imaging signals output from the camera unit 43 via the signal cable 45 (refer to Fig. 3), and performs various kinds of image processing to generate image data. The image data generated by the processor device 11 is displayed on a monitor 26 cable-connected to the processor device 11 as an observation image.

As illustrated in Fig. 2, the distal end portion 16a is provided with a gas/liquid supply nozzle 27 serving as a fluid jet nozzle. A gas/liquid supply tube 28 and a treatment tool insertion tube 29 are disposed inside the insertion portion 16 and the hand operating portion 17. The gas/liquid supply tube 28 communicates with the gas/liquid supply nozzle 27 at one end thereof. The other end of the gas/liquid supply tube 28 branches into a gas supply conduit 28a and a liquid supply conduit 28b via a merging member 70 to be described below. The gas supply conduit 28a and the liquid supply conduit 28b are connected to a cylinder 20a of the gas/liquid supply button 20 provided in the hand operating portion 17.

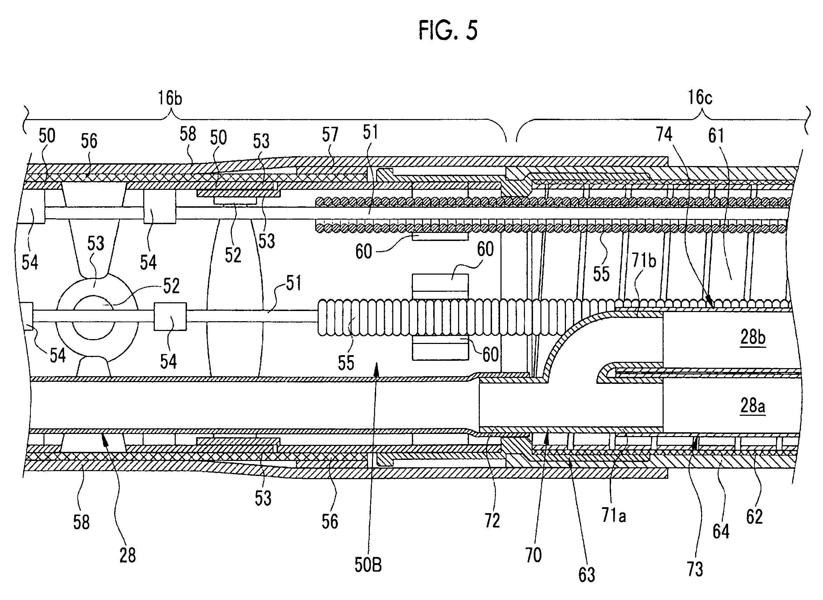

The gas/liquid supply button 20 is equipped with the cylinder 20a, a piston 20b, and an operation cap 20c. The other end of a gas supply tube 73 (refer to Fig. 5) having the gas supply conduit 28a and the other end of a liquid supply tube 74 (refer to Fig. 5) having the liquid supply conduit 28b are connected to the cylinder 20a. One end of a gas supply source conduit 30 that leads to a gas supply device 13a and one end of a liquid supply source conduit 31 that leads to the liquid tank 13b are connected to the cylinder 20a. The gas supply device 13a continuously supplies gas during endoscopy using the electronic endoscope 10. The piston 20b is slidable with respect to the cylinder 20a, and the operation cap 20c is coupled to one end that protrudes from the cylinder 20a. The operation cap 20c is formed with a leakage hole (not illustrated).

The gas supply source conduit 30 and the external atmospheric air communicate with each other via the leakage hole in a state where surgeon's fingers do not come into contact with the operation cap 20c. If a gas supply operation is performed with the leakage hole being in a blocked state, the gas supply conduit 28a and the gas supply source conduit 30 communicate with each other via a first communication path within the piston 20b. Accordingly, the gas generated from the gas supply device 13a is supplied to the gas/liquid supply tube 28 via the gas supply source conduit 30, the gas/liquid supply button 20, and the gas supply conduit 28a. Additionally, if the operation cap 20c is pushed in to perform a liquid supply operation, an outer peripheral surface of the piston 20b interrupts the communication between the gas supply conduit 28a and the gas supply source conduit 30, and the liquid supply source conduit 31 and the liquid supply conduit 28b communicate with each other via a second communication path in the piston 20b. Accordingly, cleaning water is supplied into the gas/liquid supply tube 28 via the liquid supply source conduit 31, the gas/liquid supply button 20, and the liquid supply conduit 28b from the liquid tank 13b due to the pressure of the gas generated from the gas supply device 13a. The gas/liquid supply nozzle 27 selectively jets the gas and the cleaning water therefrom, which are supplied via the gas/liquid supply tube 28, through the switching operation of the gas/liquid supply button 20.

One end of the treatment tool insertion tube 29 communicates with a treatment tool outlet 32 and the other end thereof is connected to the treatment tool inlet 19. Various treatment tools having an injection needle, a high-frequency knife, and the like disposed at the distal ends thereof are inserted into the treatment tool inlet 19, and the treatment tool inlet is blocked by a plug (not illustrated) except when a treatment tool is inserted therein. Additionally, a suction conduit 33 branches from the treatment tool insertion tube 29, and the suction conduit 33 is connected to a cylinder 21a of the suction button 21.

The suction button 21 is equipped with the cylinder 21a, a piston 21b, and an operation cap 21c. The other end of a suction source conduit 34 other than the suction conduit 33 is connected the cylinder 21a. The suction device 14 continuously operates during inspection using the electronic endoscope 10. The piston 21b is slidable with respect to the cylinder 21a, and the operation cap 21c is coupled to one end that protrudes from the cylinder 21a. If the operation cap 21c is pushed to perform a suction operation, the suction conduit 33 and the suction source conduit 34 communicate with each other via a communication path within the piston 21b. Accordingly, the negative pressure suction force of the suction conduit 33 and the treatment tool insertion tube 39 rises, and suction is performed from the treatment tool outlet 32. Additionally, if the interruption operation of releasing the operation cap 21c which is pushed-in is performed, an outer peripheral surface of the piston 21b interrupts the communication between the suction conduit 33 and the suction source conduit 34, and the suction from the treatment tool outlet 32 stops.

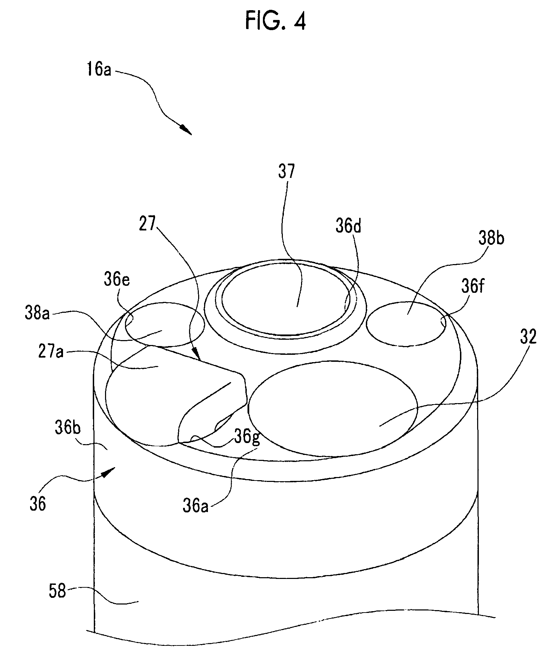

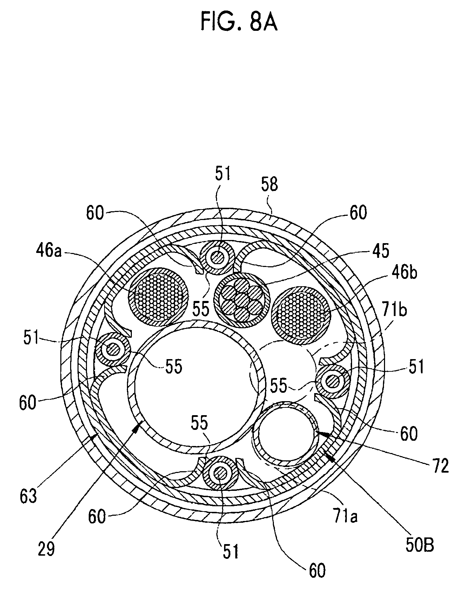

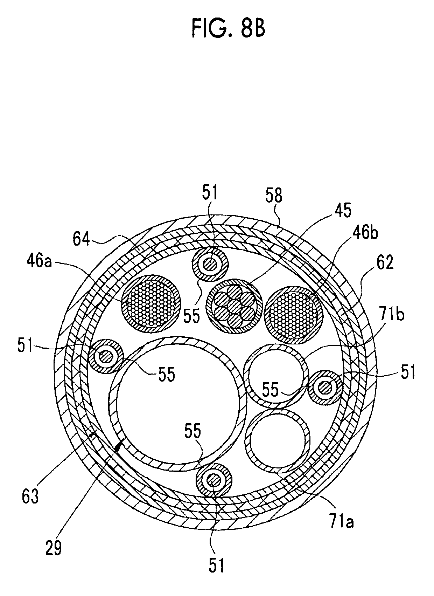

As illustrated in Fig. 3, the distal end portion 16a is equipped with a distal end portion main body 35, a distal end cap 36 having a cap-like shape that is mounted on a distal end side of this distal end portion main body 35, an observation window 37, two illumination windows 38a and 38b (refer to Fig. 4), the gas/liquid supply nozzle 27, and the treatment tool outlet 32 (refer to Fig. 4). In the distal end portion main body 35, through-holes 35a and 35b that hold respective parts, such as an objective lens unit 41, the gas/liquid supply nozzle 27, a connection pipe 48, and light guides 46a and 46b (refer to Figs. 8A and 8B), are formed along the axial direction of the insertion portion 16. A proximal end of the distal end portion main body 35 is coupled to a distal end piece 50A, which is one of the bendable pieces 50 that constitutes the bendable portion 16b and is located on the most distal end side of the bendable portion 16b. In addition, in Figs. 3 and 5, the treatment tool insertion tube 29, the light guides 46a and 46b, and the like are omitted in order to prevent complication of the drawings.

The distal end cap 36 has a distal end plate portion 36a that covers a distal end side of the distal end portion main body 35, and a cylinder portion 36b that covers an outer peripheral surface of the distal end portion main body 35. An angled rubber piece 58 that covers an outer peripheral surface of the bendable portion 16b extends up to the distal end portion main body 35, a distal end of the angled rubber piece 58 and a proximal end of the cylinder portion 36b are butted against each other, and these ends are anchored to each other with an adhesive or the like.

As illustrated in Fig. 4, the distal end plate portion 36a is formed with the observation window 37, the illumination windows 38a and 38b, through-holes 36d to 36g that exposes the gas/liquid supply nozzle 27, and the treatment tool outlet 32. As illustrated in Fig. 3, the observation window 37 also serves as a cover glass of the camera unit 43, and is an objective lens provided on the most distal end side of the objective lens unit 41. An optical system of the objective lens unit 41 including the observation window 37 is held by a lens barrel 42. The lens barrel 42 holds a proximal end side of an outer peripheral surface of the observation window 37. A distal end side of the outer peripheral surface of the observation window 37 is fitted to a through-hole 36d of the distal end cap 36.

The lens barrel 42 is fitted to the through-hole 35a of the distal end portion main body 35, and a distal end surface thereof strikes the distal end plate portion 36a of the distal end cap 36, and is disposed at a position where the observation window 37 is exposed from the distal end side of the distal end plate portion 36a. In addition, the observation window 37 may be a glass cover that is located on the most distal end side of the objective lens unit 41 and has no lens effect. In addition, the observation window 37 may not constitute the objective lens unit 41, and alternatively it may be fitted and fixed to the through-hole 36d of the distal end cap 36 as a mere glass cover.

The objective lens unit 41 consists of an objective lens or a prism 41a, and the optical axis of the optical system is bent at 90 degrees by the prism 41 a. The imaging element 43a is transversely arranged so that an emission surface, through which image light is emitted from the prism 41 a, and an imaging surface, face each other. A circuit board 44 is mounted with the imaging element 43a and is also mounted with a drive circuit that drives the imaging element 43a. The signal cable 45 consisting of a multi-core cable is connected to the circuit board 44. The signal cable 45 extends from the insertion portion 16 through the hand operating portion 17 to the universal cord 18, and is connected to the processor device 11. The imaging element 43a consists of, for example, an interline transfer type charge coupled device (CCD), and a subject image taken in by the optical system of the objective lens unit 41 is focused on the imaging surface. In addition, the imaging element 43a is not limited to the CCD, and a complementary metal oxide Semiconductor (CMOS) or other devices may be used.

The illumination windows 38a and 38b also serve as irradiation lenses, and allow a region to be observed within a living body to be irradiated with the illumination light from the light source device 12 therethrough. Emission ends of the light guides 46a and 46b (refer to Figs. 8A and 8B) face the illumination windows 38a and 38b. The light guides 46a and 46b are formed by bundling a number of optical fibers together. The light guides 46a and 46b guide the illumination light from the light source device 12 to the illumination windows 38a and 38b through the inside of the insertion portion 16, the hand operating portion 17, the universal cord 18, and the connector 24. In addition, the light guided from the light source device 12 may be, for example, excitation light, such as laser light. In this case, a system is preferably used which guides the excitation light from the light source device 12 with a single-line optical fiber, makes a fluorescent body disposed at the distal end portion 16a emit light, and radiates illumination light.

The gas/liquid supply nozzle 27 is connected to the connection pipe 48 via the gas/liquid supply tube 28. The connection pipe 48 is fitted to and held by the through-hole 35b of the distal end portion main body 35. A proximal end of the gas/liquid supply nozzle 27 is fitted to an outer peripheral surface of one end of the connection pipe 48, and one end of the gas/liquid supply tube 28 is fitted to the other end of the connection pipe 48. A jet tube portion 27a is formed on a distal end side of the gas/liquid supply nozzle 27. The jet tube portion 27a is formed in a tubular shape protruding in a direction in which the jet tube portion 27a is bent, for example, at 90 degrees from the proximal end of the gas/liquid supply nozzle 27, and has a jet port 27b at the distal end thereof.

The bendable portion 16b has a plurality of (for example, sixteen) bendable pieces 50 that are coupled in series. In the following, for the sake of description, a bendable piece located on the most distal side of the bendable portion 16b among the plurality of bendable pieces 50 is referred to as a distal end piece 50A, and a bendable piece located on the most proximal end side of the bendable portion 16b is referred to as a proximal end piece 50B. The distal end piece 50A is fixed to the distal end portion main body 35, and the proximal end piece 50B is fixed to the flexible portion 16c. A bendable operation wire 51 (refer to Figs. 3 and 5) extending from the hand operating portion 17 through the flexible portion 16c to the bendable portion 16b is coupled to the bendable pieces 50.

Two up-and-down and right-and-left bendable operation wires 51 are provided, and the proximal end sides of the respective bendable operation wires 51 are hung around pulleys that rotate while interlocking with the operation of an up-and-down bendable operation knob 22 and a left-and-right bendable operation knob 23 that are provided for the hand operating portion 17. If the up-and-down bendable operation knob 22 is rotationally operated, the up-and-down bendable operation wire 51 is pushed and pulled and the bendable portion 16b is operated in a bendable manner in an up-down direction, and if the left-and-right bendable operation knob 23 is rotationally operated, the bendable operation wire 51 is pushed and pulled and the bendable portion 16b is operated in a bendable manner in a left-right direction. Accordingly, the distal end portion 16a can be directed in a desired direction within a living body.

As illustrated in Fig. 5, the bendable pieces 50 are made of metal, and the plurality of bendable pieces 50 are coupled together by coupling pins 52 each used as a rotation center of each bendable piece 50. The proximal end piece 50B is coupled to a coupling ring 63 of the flexible portion 16c. Except for the distal end piece 50A and the proximal end piece 50B, a pair of tongue pieces 53 through which the coupling pins 52 are inserted are provided on a distal end side and a rear end side, respectively, of each bendable piece 50. The tongue pieces 53 are alternately provided on the upper and lower and left and right sides such that the tongue pieces are provided on the left and right sides of the proximal end side when the tongue pieces are provided the upper and lower portions of the distal end side. In the bendable pieces 50 adjacent to each other, the upper and lower or left and right tongue pieces 53 are coupled together via the coupling pin 52.

Additionally, wire guides 54 that guide the two up-and-down and right-and-left bendable operation wires 51 are formed on inner peripheral surfaces of the bendable pieces 50 excluding the proximal end piece 50B within the bendable portion 16b. Additionally, a coil pipe 55 serving as a guide member that guides each bendable operation wire 51 is provided within the flexible portion 16c.

The bendable operation wire 51 is slidably inserted through the wire guides 54. The distal end of the bendable operation wire 51 is fixed to the distal end piece 50A by brazing or the like, and the proximal end side thereof is provided so as to extend to the hand operating portion 17 and is attached to the pulley. If the up-and-down bendable operation knob 22 is rotationally operated, one set of up-and-down bendable operation wire 51 is pushed and pulled, each bendable piece 50 rotates about the coupling pin 52 attached to the left and right tongue pieces 53, and the bendable portion 16b is bent in the up-down direction. If the left-and-right bendable operation knob 23 is rotationally operated, one left-and-right bendable operation wire 51 is pushed and pulled, each bendable piece 50 rotates about the coupling pin 52 attached to the upper and lower tongue pieces 53, and the bendable portion 16b is bent in the left-right direction.

The signal cable 45, the light guides 46a and 46b, the gas/liquid supply tube 28, and the treatment tool insertion tube 29 (refer to Figs. 8A and 8B) are inserted within the bendable pieces 50. Outer peripheries of the bendable pieces 50 are covered with a braid 56. A metal ring 57 is fitted to an outer peripheral surface of the braid 56. In addition, in Figs. 3 and 5, the metal ring 57 is provided only at a proximal end of the braid 56. However, the metal ring 57 may be provided at a distal end portion of the braid 56. An outer periphery of the braid 56 is covered with an angled rubber piece 58, which is an outer cover that is formed in a tubular shape and made of rubber. The braid 56 is a net-like body formed by braiding a plurality of metallic element wires together. The braid 56 covers the plurality of coupled bendable pieces 50, thereby stabilizing the posture of each bendable piece 50. Additionally, an inner peripheral surface of the angled rubber piece 58 comes in contact with the braid 56, and adhesion force is enhanced.

A distal end side of the angled rubber piece 58 also covers the distal end portion main body 35 of the distal end portion 16a. The distal end of the angled rubber piece 58 has thread (not illustrated), for example, wound therearound and is fixed to the distal end portion 16a. A seal material or an adhesive are coated and is hardened on a portion where the thread is wound. Additionally, a proximal end side of the angled rubber piece 58 is covered to the distal end side of the flexible portion 16c.

Fixing portions 60 are provided inside the proximal end piece 50B. Each fixing portion 60 is a bent portion formed by bending a portion of a cylinder portion of the proximal end piece 50B inward, pinches an outer peripheral surface of the coil pipe 55, and fixes the coil pipe to the proximal end piece 50B. In addition, the fixing portion 60 may have a configuration in which the coil pipe 55 is fixed using a lock pin or the like.

The flexible portion 16c has a spiral tube 61 that is formed by spirally winding a strip made of metal, such as stainless steel, a braid 62 that is a net-like body that is put on the outer periphery of the spiral tube 61 and formed by braiding an element wire made of metal, such as stainless steel, in the shape of a net, coupling rings 63 that are made of metal, such as stainless steel, and anchored to outer peripheral surfaces of both ends of the braid 62, and an outer cover 64 that is covered on an outer periphery of the braid 62 by extrusion molding and is made of resin. The coupling rings 63 are provided on the most distal end side and the most proximal end side of the flexible portion 16c. The coupling ring 63 on the distal end side is coupled to the bendable portion 16b, and the coupling ring 63 on the proximal end side is coupled to the hand operating portion 17. The coupling ring 63 is fitted to the outer periphery of the braid 62, and a portion thereof is covered with the outer cover 64 together with the braid 62. In addition, as the configuration of the flexible portion 16c, the braid 62 may be eliminated, and an outer peripheral surface of the spiral tube 61 may be directly covered with the outer cover 64.

An outer peripheral surface of the proximal end piece 50B is fitted to an inner peripheral surface of the coupling ring 63 on the distal end side. In the assembly process of coupling the bendable portion 16b and the flexible portion 16c together, the outer peripheral surface of the proximal end piece 50B is fitted to the inner peripheral surface of the coupling ring 63, and the proximal end piece 50B and the coupling ring 63 are joined together by soldering or the like. In addition, a method of joining the proximal end piece 50B and the coupling ring 63 is not limited to the soldering, and joining methods, such as screw fastening and engaging metal parts with each other, may be adopted.

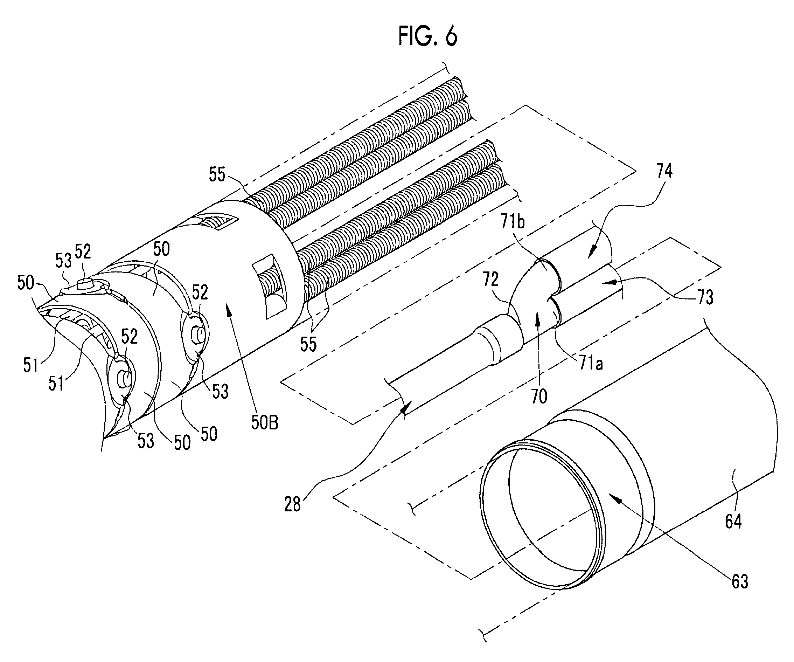

The merging member 70 is disposed inside the insertion portion 16. As illustrated in Fig. 6, the merging member 70 has branch pipe portions 71a and 71b and a merging pipe portion 72. The branch pipe portions 71a and 71b are constituted by the proximal end portion of the merging member 70 branched out into two. An inner peripheral surface of the gas supply tube 73 having the gas supply conduit 28a is fitted to an outer peripheral surface of the branch pipe portion 71a. An inner peripheral surface of the liquid supply tube 74 having the liquid supply conduit 28b is fitted to an outer peripheral surface of the branch pipe portion 71b. Accordingly, the gas supply tube 73 and the liquid supply tube 74 communicate with the branch pipe portions 71a and 71b. In addition, in Fig. 6, the braid 56, the metal ring 57, the angled rubber piece 58, and the like are omitted in order to prevent complication of the drawing.

The merging pipe portion 72 is provided continuously with distal end sides of the branch pipe portions 71a and 71b. The merging pipe portion 72 has an inner peripheral surface of the gas/liquid supply tube 28 fitted to an outer peripheral surface thereof, and communicates with the gas/liquid supply tube 28. Accordingly, the merging member 70 communicates with the gas/liquid supply nozzle 27 via the connection pipe 48 and the gas/liquid supply tube 28. The merging pipe portion 72 is formed linearly with one branch pipe portion 71a. The other branch pipe portion 71b is joined to outer peripheral surfaces of the merging pipe portion 72 and the other branch pipe portion 71b and is bent at 90 degrees, and a proximal end of the branch pipe portion 71b is disposed parallel to the merging pipe portion 72 and the branch pipe portion 71a. Tubes having flexibility and collapse resistance are used as the gas/liquid supply tube 28, the gas supply tube 73, and the liquid supply tube 74.

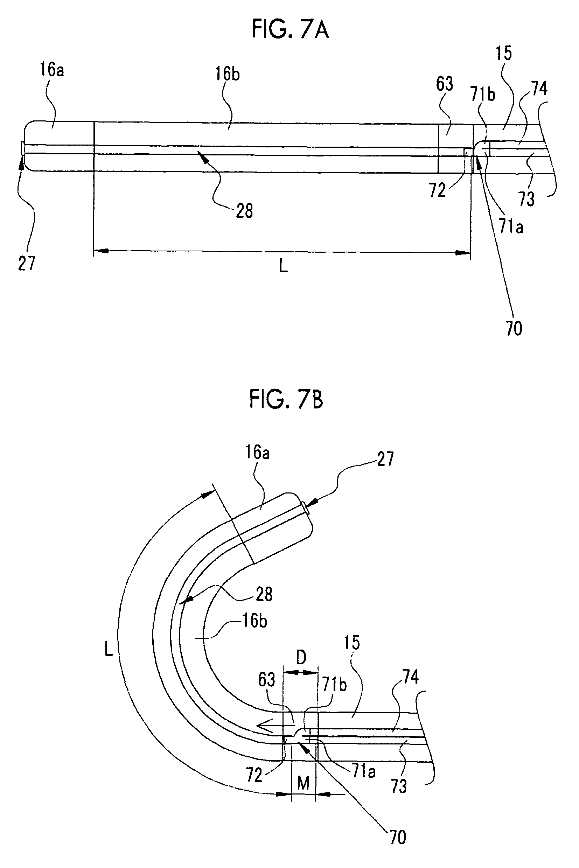

The position of the merging member 70 within the insertion portion 16 is determined depending on the length of the gas/liquid supply tube 28. As illustrated in Fig. 7A, the bendable portion 16b is in a linear state in which the gas/liquid supply tube 28 is linear. Then, in the merging member 70 that communicates with the other end of the gas/liquid supply tube 28 in the linear state, both the branch pipe portions 71a and 71b and the merging pipe portion 72 may be disposed within the flexible portion 16c, a distal end of the merging pipe portion 72 may be disposed within the coupling ring 63, and a portion of the merging pipe portion 72 and the branch pipe portions 71a and 71b may be disposed within the flexible portion 16c. Then, as illustrated in Fig. 7B, if the bendable portion 16b is brought into a maximally bent state by the operation of the bendable operation knobs 22 and 23, the gas/liquid supply tube 28 is bent together with the bendable portion 16b. In addition, the maximally bent state here means a state where the curvature radius of the bendable portion 16b is minimum when the bendable portion is operated in a bendable manner in any one of the up-down direction and the left-right direction. The merging member 70 that communicates with the other end of the gas/liquid supply tube 28 that is brought into this maximally bent state is pulled in to the distal end side of the insertion portion 16. Reference sign M represents the movement amount of the merging member 70 pulled in by the gas/liquid supply tube 28 that is brought into the maximally bent state together with the bendable portion 16b. Also, the gas/liquid supply tube 28 has a length L such that the merging pipe portion 71 stays within the coupling ring 63 when the merging member 70 is pulled in at a movement amount M by bending the bendable portion 16b. In addition, the state "stays in the coupling ring 63" here may be any position as long as the merging pipe portion is inside the coupling ring 63, or may be within a proximal end of the proximal end piece 50B fitted to the inner peripheral surface of the coupling ring 63. Additionally, even a portion of the merging pipe portion 72 only has to stay within the coupling ring 63, or even if the distal end side of the merging pipe portion 72 enters the proximal end piece 50B, a portion of the merging pipe portion 72 only has to stay within the coupling ring 63.

As a specific configuration of the gas/liquid supply tube 28 and the merging member 70, if the length L of the gas/liquid supply tube 28 is, for example, 65 mm or more and 100 mm or less, the movement amount M by which the merging member 70 is pulled in is 3 mm or more and 10 mm or less. The length D of the cylinder center direction of the coupling ring 63 is, for example, 15 mm. In this case, when the bendable portion 16b is in the linear state, the movement amount M is smaller than the length D of the coupling ring 63 even if the distal end of the merging pipe portion 72 is at the same position as a proximal end surface of the coupling ring 63 or a portion of the merging pipe portion is within the coupling ring 63. Therefore, the merging pipe portion 72 stays within the coupling ring 63.

The position of the merging member 70 is determined depending on the length L of the gas/liquid supply tube 28 as described above, and the position of the merging member is regulated depending on built-in elements within the bendable portion 16b and the flexible portion 16c. As illustrated in Fig. 8A, built-in elements inserted through the proximal end piece 50B include the signal cable 45, the light guides 46a and 46b, the treatment tool insertion tube 29, the coil pipes 55 through which the bendable operation wires 51 are inserted, the fixing portions 60, and the gas/liquid supply tube 28. The coil pipes 55 and the fixing portions 60 are disposed at positions running along an inner peripheral surface of the proximal end piece 50B.

Since the external diameter of the treatment tool insertion tube 29 among the built-in elements within the proximal end piece 50B is greater than the spacing between the fixing portions 60, the treatment tool insertion tube 29 abuts against the fixing portions 60, and is thereby disposed at a position apart from the inner peripheral surface of the proximal end piece 50B and near the center. Additionally, since the light guides 46a and 46b and the gas/liquid supply tube 28 have external diameters smaller than the spacing between the fixing portions 60, the light guides and the gas/liquid supply tube are disposed along the inner peripheral surface of the proximal end piece 50B without abutting against the fixing portions 60. Additionally, although the signal cable 45 has an external diameter which is smaller than the spacing between the fixing portions 60, the signal cable is at a position where the signal cable is sandwiched by the light guides 46a and 46b. Therefore, the signal cable abuts against the fixing portions 60, and is disposed at a position where the signal cable is separated from the inner peripheral surface of the proximal end piece 50B and abuts on the treatment tool insertion tube 29. As the fixing portions 60 are disposed and the signal cable 45 and the treatment tool insertion tube 29 are disposed at the positions near the center, the inside of the proximal end piece 50B is provided with a space into which the merging pipe portion 72 or the gas/liquid supply tube 28 of which inner peripheral surface is fitted to the outer peripheral surface the merging pipe portion 72 enters. However, the branch pipe portions 71a and 71b having a cross-sectional area that is twice as large as this space or the gas supply tube 73 and the liquid supply tube 74 of which inner peripheral surfaces are fitted to the outer peripheral surfaces of the branch pipe portions 71a and 71b respectively cannot be arranged in this space. In addition, two-point chain lines in Fig. 8A illustrate cross-sections of the branch pipe portions 71a and 71b, and the entry of the branch pipe portions 71a and 71b into the proximal end piece 50B is regulated due to abutment against the treatment tool insertion tube 29, the light guides 46a and 46b, the fixing portions 60, or the like.

As illustrated in Fig. 8B, built-in elements to be inserted through the flexible portion 16c include the signal cable 45, the light guides 46a and 46b, the treatment tool insertion tube 29, the coil pipes 55 through which the bendable operation wires 51 are inserted, the branch pipe portions 71a and 71 b or the gas supply tube 73, and the liquid supply tube 74. There is a margin for a packing ratio with respect to the inside of the proximal end piece 50B as long as there are no fixing portions 60 at a position closer to the proximal end side than the proximal end piece 50B inside the flexible portion 16c or the coupling ring 63, and the treatment tool insertion tube 29 and the signal cable 45 can be arranged near an inner peripheral surface of the flexible portion or the coupling ring. For this reason, the branch pipe portions 71a and 71b or the gas supply tube 73, and the liquid supply tube 74 can be arranged inside the flexible portion 16c or the coupling ring 63.

The operation of the above configuration will be described. After inspection preparation of the endoscope system 2 is completed, the imaging element 43a operates, and gas supply using the gas supply device 13a and suction using the suction device 14 are continuously performed. Then, the insertion portion 16 is inserted into the inside of a body, for example, an alimentary canal after the completion of the preparation. The light from the light source device 12 is radiated to a region to be observed within the alimentary canal through the light guides 46a and 46b within the universal cord 18 and the insertion portion 16 and the illumination windows 38a and 38b of the distal end portion 16a. The imaging element 43a within the distal end portion 16a images the inside of the alimentary canal, and outputs imaging signals. The imaging signals are input to the processor device 11 via the signal cable 45 within the insertion portion 16, and the universal cord 18, and are displayed on the monitor 26.

When dirt has adhered to the observation window 37, cleaning water is jetted from the jet port 27b of the gas/liquid supply nozzle 27 by the liquid supply operation of the gas/liquid supply button 20, and cleans the observation window 37. After the cleaning of the observation window 37, air is further jetted from the jet port 27b by the gas supply operation of the gas/liquid supply button 20, and the cleaning water that has remained on the observation window 37 is blown out. As described above, the gas/liquid supply tube 28 has a length L such that the merging member 70 stays within the coupling ring when the merging member 70 is pulled in at the movement amount M by the bending of the bendable portion 16b. That is, the length L of the gas/liquid supply tube 28 can be sufficiently shortened, and waterdrops that have adhered to the gas/liquid supply tube 28 after the liquid supply of the cleaning water is immediately blown out by the air that has been supplied, and the cleaning water within the gas/liquid supply tube 28 can be prevented from remaining therein. Accordingly, droplets can be jetted from the gas/liquid supply nozzle 27 together with gas during a gas supply operation and can be prevented from adhering to the observation window 37.

Meanwhile, when the inside of the body is observed using the endoscope system 2, the bendable portion 16b may be bent and the orientation of the distal end portion 16a may be changed. As described above, even if the bendable portion 16b is brought into the maximally bent state, the merging pipe portion 72 stays within the coupling ring 63, and the entry of the branch pipe portions 71a and 71b into the proximal end piece 50B is regulated. For this reason, although the gas/liquid supply tube 28 or the merging pipe portion 72 enters the proximal end piece 50B having a high packing ratio, the entry therein of the branch pipe portions 71a and 71b or the gas supply tube 73, and the liquid supply tube 74 can be prevented. Since the cross-sectional area of the gas/liquid supply tube 28 or the merging pipe portion 72 is smaller than the total cross-sectional area of the branch pipe portions 71a and 71 b or the total cross-sectional area of the gas supply tube 73 and the liquid supply tube 74, the packing ratio within the proximal end piece 50B is not raised. This does not hinder the bending of the bendable portion 16b.

### <Second Embodiment>

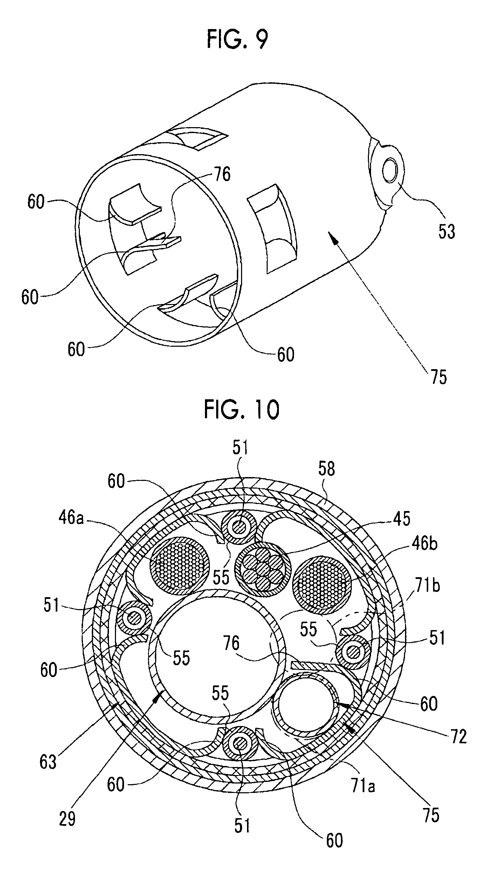

An example in which the positions of the branch pipe portions 71a and 71b are regulated depending on the arrangement of built-in elements, such as the signal cable 45, the light guides 46a and 46b, the treatment tool insertion tube 29, the coil pipes 55, and the fixing portions 60 is described in the above first embodiment. However, the invention is not limited to this, and protruding portions 76 made to protrude from each fixing portion 60 toward the merging member 70 may be integrally formed as in a proximal end piece 75 that constitutes an insertion portion of a second embodiment illustrated in Fig. 9. Since the built-in elements within the insertion portion 16 move slightly depending on the bending of the bendable portion 16b or the insertion shape of the flexible portion 16c, a space where the branch pipe portions 71a and 71b can enter the proximal end piece 50B may be formed in the above first embodiment. However, in the second embodiment, as illustrated in Fig. 10, the protruding portions 76 are made to abut against the branch pipe portion 71b, and thereby, the entry of the branch pipe portions 71a and 71b into the proximal end piece 50B is reliably regulated.

### <Third Embodiment>

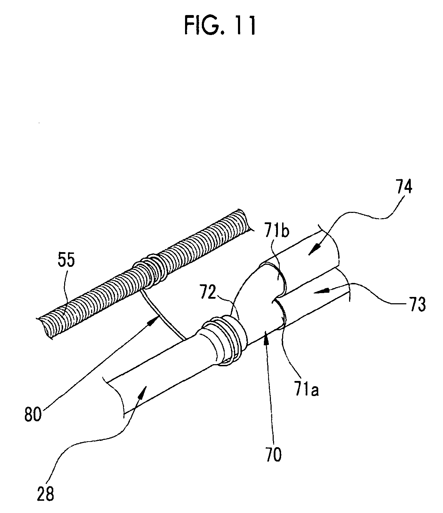

Additionally, as another example in which the position of the branch pipe portions 71a and 71b is regulated, as in a third embodiment illustrated in Fig. 11 a spring 80 serving as a biasing member may be provided between the coil pipe 55 and the merging member 70. In this case, the spring 80 is attached by winding one end around the coil pipe 55 and winding the other end around the merging pipe portion 72. Accordingly, when the merging member 70 is pulled in by bending the bendable portion 16b, the spring 80 biases the merging member 70 to a proximal end side of the flexible portion 16c. Through the biasing of this spring 80, the entry of the branch pipe portions 71a and 71b into the proximal end piece 50B can be regulated, and the position of the merging member 70 can be returned when the bendable portion 16b is brought into the linear state. In addition, when attaching the spring 80 to the coil pipes 55 and the merging member 70, other attaching methods, such as brazing and soldering, may be used. Additionally, the third embodiment may be a combination of the above first and second embodiments. That is, the fixing portions 60 of the above first embodiment or the protruding portions 76 of the second embodiment may regulate the positions of the branch pipe portions 71a and 71b, and the spring 80 may bias the merging member 70.

In the above respective embodiments, an example is shown in which the other branch pipe portion 71b is joined to the outer peripheral surface of the branch pipe portion 71a and while it is linearly formed with the merging pipe portion 72, is bent at 90 degrees, and is described as the merging member 70. However, the invention is not limited to this. A Y-shaped merging member may be formed in which a pair of branch pipe portions are bent at the same angle with respect to the merging pipe portion and the branch pipe portions are disposed in directions symmetrical to each other.

In the above respective embodiments, the merging member 70, the gas/liquid supply tube 28, the gas supply tube 73, and the liquid supply tube 74 are provided as separate members, respectively, and connected to each other. However, the merging member 70, the gas/liquid supply tube 28, the gas supply tube 73, and the liquid supply tube 74 may be integrally formed. That is, the gas/liquid supply tube 28 is formed integrally with the distal end side of the merging pipe portion, and the gas supply tube 73 and the liquid supply tube 74 are formed integrally with the proximal end sides of the branch pipe portions. Additionally, even in this case, even a portion of the merging pipe portion may stay within the coupling ring 63 when the merging member is pulled in by bending the bendable portion 16b. Additionally, in the above respective embodiments, the gas supply tube 73 and the liquid supply tube 74 are directly connected to the cylinder 20a. However, the invention is not limited to this. The gas supply tube 73 and the liquid supply tube 74 may be connected to the cylinder 20a via a separate tube.

In the above respective embodiments, the electronic endoscope for observing the inside of the body of a subject creates an image that is captured using the imaging element is described as an example. However, the invention is not limited to this and can also be applied to an endoscope in which an optical image guide is adopted to observe the state of the inside of the body of a subject.

Although the preferable embodiments of the invention have been described above in detail, the invention is not limited to the above-described specific embodiments, and various alterations and changes can be made within the scope of the invention described in the claims.

## Claims

1. An endoscope insertion portion (16) comprising: a distal end portion (16a); a bendable portion (16b); and a flexible portion (16c), sequentially from a distal end from the endoscope insertion portion (16), the endoscope insertion portion (16) further comprising:
a fluid jet nozzle (27) that is provided at the distal end portion (16a), the jet nozzle (27) being adapted to jet a gas and a liquid therefrom;
a gas supply tube (73) and a liquid supply tube (74) adapted to supply the gas and the liquid respectively from a gas/liquid supply device (13) provided outside the endoscope insertion portion (16);
a plurality of bendable pieces (50) that are provided at the bendable portion (16b) and coupled together in series;
a coupling ring (63) that is provided on a most distal end side of the flexible portion (16c) and is coupled to a proximal end piece (50B), which is a bendable piece provided on a most proximal end side among the bendable pieces (50);
a merging member (70) comprising: branch pipe portions (71 a; 71 b) that are constituted by a proximal end of the merging member branched apart and communicate with the gas supply tube (73) and the liquid supply tube (74), respectively; and a merging pipe portion (72) that is provided continuously with the branch pipe portions;
a gas/liquid supply tube (28) that allows the fluid jet nozzle (27) and the merging pipe portion to communicate with each other; and
a built-in element (29) to be inserted through the bendable portion (16b) and the flexible portion (16c) together with the merging member,
wherein the proximal end piece has therein a space where the merging pipe portion or the gas/liquid supply tube (28) fitted an outer peripheral surface of the merging pipe portion enters **characterised in that** the gas/liquid supply tube has a length such that at least a portion of the merging pipe portion (72) stays in the coupling ring (63) when the merging member is pulled in to the distal end portion by the bendable portion being bent, and an entry of the branch pipe persons (71a; 71 b) into the proximal end piece is prevented due to abutment of the branch pipe portions (71 a; 71b) against the built-in element (29).

2. The endoscope insertion portion (16) according to claim 1, wherein a part of the merging pipe portion is located in the flexible portion (16c) in a state where the bendable portion (16b) is in a linear state.

3. An endoscope insertion portion (16) comprising: a distal end portion (16a); a bendable portion (16b); and a flexible portion (16c), sequentially from a distal end from the endoscope insertion portion (16), the endoscope insertion portion (16) further comprising:
a fluid jet nozzle (27) that is provided at the distal end portion (16a), the jet nozzle (27) being adapted to jet a gas and a liquid therefrom;
a gas supply tube (73) and a liquid supply tube (74) adapted to supply the gas and the liquid respectively from a gas/liquid supply device (13) provided outside the endoscope insertion portion (16);
a plurality of bendable pieces (50) that are provided at the bendable portion (16b) and coupled together in series;
a coupling ring (63) that is provided on a most distal end side of the flexible portion (16c) and is coupled to a proximal end piece (50B), which is a bendable piece provided on a most proximal end side among the bendable pieces (50);
a merging member (70) comprising: branch pipe portions (71a; 71b) that are constituted by a proximal end of the merging member branched apart and communicate with the gas supply tube (73) and the liquid supply tube (74), respectively; and a merging pipe portion (72) that is provided continuously with the branch pipe portions;
a gas/liquid supply tube (28) that allows the fluid jet nozzle (27) and the merging pipe portion to communicate with each other; and
a bendable operation wire (51) adapted to operate the bendable portion in a bending manner **characterised in that** the gas/liquid supply tube (28) has a length such that at least a portion of the merging pipe portion (72) stays in the coupling ring (63) when the merging member is pulled in to the distal end portion by the bendable portion being bent, and by
a guide member (55) adapted to guide the bendable operation wire (51); and
a fixing portion (60) that is provided inside the proximal end piece and is adapted to the guide member,
wherein the entry of the branch pipe portion 71a; 71b) into the proximal end piece is prevented due to abutment of the branch pipe portion (71a; 71b) against the fixing portion (60).

4. The endoscope insertion portion (16) according to claim 3,
wherein a part of the merging pipe portion is located in the flexible portion (16c) in a state where the bendable portion (16b) is in a linear state.

5. The endoscope insertion portion (16) according to claim 3 or 4,
wherein the fixing portion is constituted by a bent portion that is formed by bending a part of a cylinder portion of the proximal end piece (50B) inward, the fixing portion having a protruding portion (76) that is formed integrally with the bent portion and is made to protrude toward the merging member, and
wherein the fixing portion (60) prevents the entry of the branch pipe portion (71a; 71b) into the proximal end piece due to abutment of the branch pipe portion against the protruding portion (76).

6. The endoscope insertion portion (16) according to any one of claims 3 to 5, further comprising:
a biasing member (80) that is provided between the guide member (55) and the merging member and is adapted to bias the merging member to a proximal end side of the flexible portion to prevent the entry of the branch pipe portion (71 a; 71 b) into the proximal end piece.

7. The endoscope insertion portion (16) according to any one of claims 1 to 6,
wherein the gas/liquid supply tube (28), the liquid supply tube (74), the gas supply tube (73), and the merging member are integrally formed.

8. An endoscope (10) comprising:
the endoscope insertion portion (16) according to any one of claims 1 to 7;
a hand operating portion (17) that is provided continuously with the endoscope insertion portion (16); and
a bending operation member (22, 23) adapted to performs the bending of the bendable portion (16b).

## Patentansprüche

1. Endoskopeinführabschnitt (16), umfassend:
einen distalen Endabschnitt (16a); einen biegbaren Abschnitt (16b) und einen flexiblen Abschnitt (16c), die sequentiell von einem distalen Ende des Endoskopeinführabschnitts (16) aus vorgesehen sind, wobei der Endoskopeinführabschnitt (16) weiterhin aufweist:
eine Fluidstrahldüse (27), die sich an dem distalen Endabschnitt (16a) befindet und dazu ausgebildet ist, ein Gas und eine Flüssigkeit auszustoßen;
einen Gaszuführschlauch (73) und einen Flüssigkeitszuführschlauch (74), ausgebildet zum Zuführen des Gases bzw. der Flüssigkeit aus einer Gas-/Flüssigkeitszuführeinrichtung (13), die sich außerhalb des Endoskopeinführabschnitts (16) befindet;
mehrere biegbare Stücke (50), die an dem biegbaren Abschnitt (16b) vorgesehen sind und miteinander in Reihe gekoppelt sind;
einen Kopplungsring (73), der sich an einer am weitesten distalen Endseite des flexiblen Abschnitts (16c) befindet und mit einem proximalen Endstück (50B) gekoppelt ist, bei dem es sich um ein biegbares Stück handelt, das sich an der am meisten proximalen Endseite von den biegbaren Stücken (50) befindet;
ein Übergangselement (70), umfassend: Verzweigungsrohrabschnitte (71a; 71 b), die gebildet werden durch ein proximales Ende des Übergangselements, abgezweigt von und kommunizierend mit dem Gaszuführschlauch (73) bzw. dem Flüssigkeitszuführschlauch (74); und einen Übergangsrohrabschnitt (72), der kontinuierlich fortgesetzt an die Verzweigungsrohabschnitte vorgesehen ist;
ein Gas-/Flüssigkeitszuführrohr (28), das der Fluidstrahldüse (27) und dem Übergangsrohrabschnitt ermöglicht, miteinander zu kommunizieren, und
ein Einbauelement (29) zum Einführen durch den biegbaren Abschnitt (16b) und den flexiblen Abschnitt (16c) zusammen mit dem Übergangselement,
wobei das proximale Endstück in sich einen Raum aufweist, in welchen der Übergangsrohrabschnitt oder das Gas-/Flüssigkeitszuführrohr (28), das auf einer Außenumfangsfläche des Übergangsrohrabschnitts aufgepasst ist, eintritt,
**dadurch gekennzeichnet, dass** das Gas-/Flüssigkeitszuführrohr eine derartige Länge aufweist, dass mindestens ein Teil des Übergangsrohrabschnitts (72) in dem Kopplungsring (73) verbleibt, wenn das Übergangselement durch Biegen des biegbaren Abschnitts in den distalen Endabschnitt gezogen wird, und ein Eintritt der Verzweigungsrohrabschnitte (71 a; 71 b) in das proximale Endstück verhindert wird durch das Anschlagen der Verzweigungsrohrabschnitte (71a, 71b) gegen das Einbauelement (29).

2. Endoskopeinführabschnitt (16) nach Anspruch 1,
bei dem ein Teil des Übergangsrohrabschnitts sich in dem flexiblen Abschnitt (16c) in einem Zustand befindet, in welchem sich der biegbare Abschnitt (16b) in einem geradlinigen Zustand befindet.

3. Endoskopeinführabschnitt (16), umfassend:
einen distalen Endabschnitt (16a); einen biegbaren Abschnitt (16b) und einen flexiblen Abschnitt (16c), die sequentiell von einem distalen Ende des Endoskopeinführabschnitts (16) aus vorgesehen sind, wobei der Endoskopeinführabschnitt (16) weiterhin aufweist:
eine Fluidstrahldüse (27), die sich an dem distalen Endabschnitt (16a) befindet und dazu ausgebildet ist, ein Gas und eine Flüssigkeit auszustoßen;
einen Gaszuführschlauch (73) und einen Flüssigkeitszuführschlauch (74), ausgebildet zum Zuführen des Gases bzw. der Flüssigkeit aus einer Gas-/Flüssigkeitszuführeinrichtung (13), die sich außerhalb des Endoskopeinführabschnitts (16) befindet;
mehrere biegbare Stücke (50), die an dem biegbaren Abschnitt (16b) vorgesehen sind und miteinander in Reihe gekoppelt sind;
einen Kopplungsring (73), der sich an einer am weitesten distalen Endseite des flexiblen Abschnitts (16c) befindet und mit einem proximalen Endstück (50B) gekoppelt ist, bei dem es sich um ein biegbares Stück handelt, das sich an der am meisten proximalen Endseite von den biegbaren Stücken (50) befindet;
ein Übergangselement (70), umfassend: Verzweigungsrohrabschnitte (71a; 71 b), die gebildet werden durch ein proximales Ende des Übergangselements, abgezweigt von und kommunizierend mit dem Gaszuführschlauch (73) bzw. dem Flüssigkeitszuführschlauch (74); und einen Übergangsrohrabschnitt (72), der kontinuierlich fortgesetzt an die Verzweigungsrohabschnitte vorgesehen ist;
ein Gas-/Flüssigkeitszuführrohr (28), das der Fluidstrahldüse (27) und dem Übergangsrohrabschnitt ermöglicht, miteinander zu kommunizieren, und
einen biegbaren Betätigungsdraht (51), ausgebildet zum Betätigen des biegbaren Abschnitts in biegbarer Weise,
**dadurch gekennzeichnet, dass**
das Gas-/Flüssigkeitszuführrohr (28) eine derartige Länge aufweist, dass mindestens ein Teil des Übergangsrohrabschnitts (72) in dem Kopplungsring (63) verbleibt, wenn das Übergangselement durch Biegen des biegbaren Abschnitts in den distalen Endabschnitt gezogen wird; und durch
ein Führungselement (55), ausgebildet zum Führen des biegbaren Betätigungsdrahts (51); und
einen Fixierabschnitt (60), der sich im Inneren des proximalen Endstücks befindet und an das Führungselement angepasst ist,
wobei der Eintritt des Verzweigungsrohrteils (71 a; 71 b) in das proximale Endstück verhindert wird aufgrund des Anschlagens des Verzweigungsrohrabschnitts (71a; 71 b) an dem Fixierabschnitt (60).

4. Endoskopeinführabschnitt (16) nach Anspruch 3,
bei dem ein Teil des Übergangsrohrabschnitts sich in dem flexiblen Abschnitt (16c) in einem Zustand befindet, in welchem der biegbare Abschnitt (16b) sich in einem geradlinigen Zustand befindet.

5. Endoskopeinführabschnitt (16) nach Anspruch 3 oder 4,
bei dem der Fixierabschnitt gebildet wird durch einen gebogenen Abschnitt, der gebildet wird durch Biegen eines Teils eines Zylinderabschnitts des proximalen Endstücks (50B) nach innen, wobei der Fixierabschnitt einen vorspringenden Abschnitt (76) aufweist, der einstückig mit dem gebogenen Abschnitt ausgebildet ist und in Richtung des Übergangselements vorsteht, und
wobei der Fixierabschnitt (60) den Eintritt des Verzweigungsrohrabschnitts (71a; 71 b) in das proximale Endstück verhindert durch das Anschlagen des Verzweigungsrohrabschnitts gegen den vorstehenden Abschnitt (76).

6. Endoskopeinführabschnitt (16) nach einem der Ansprüche 3 bis 5, weiterhin umfassend:
ein zwischen dem Führungselement (55) und dem Übergangselement vorgesehenes Vorspannelement (80) zum Vorspannen des Übergangselements zu der proximalen Endseite des flexiblen Abschnitts hin, um den Eintritt des Verzweigungsrohrabschnitts (71 a; 71 b) in das proximale Endstück zu unterbinden.

7. Endoskopeinführabschnitt (16) nach einem der Ansprüche 1 bis 6, bei dem das Gas-/Flüssigkeitszuführrohr (28), der Flüssigkeitszuführschlauch (76), der Gaszuführschlauch (73) und das Übergangselement einstückig ausgebildet sind.

8. Endoskop (10), umfassend:
den Endoskopeinführabschnitt (16) nach einem der Ansprüche 1 bis 7;
einen Handbetätigungsabschnitt (17), der sich kontinuierlich an den Endoskopeinführabschnitt (16) anschließt; und
ein Biegebetätigungselement (22, 23), ausgebildet zum Ausführen der Biegung des biegbaren Abschnitts (16b).

## Revendications

1. Portion d'introduction d'endoscope (16), comprenant : une portion d'extrémité distale (16a) ; une portion pouvant être fléchie (16b), et une portion flexible (16c), de manière séquentielle à partir d'une extrémité distale à partir de la portion d'introduction d'endoscope (16), la portion d'introduction d'endoscope (16) comprenant en outre :
une buse à jet de fluide (27), prévue au niveau de la portion d'extrémité distale (16a), la buse à jet (27) étant apte à projeter un gaz et un liquide à partir d'elle ;
un tube d'alimentation en gaz (73) et un tube d'alimentation en liquide (74) apte à alimenter le gaz et le liquide respectivement à partir d'un dispositif d'alimentation en gaz/liquide (13) prévu à l'extérieur de la portion d'introduction d'endoscope (16) ;
une pluralité de pièces pouvant être fléchies (50) prévues au niveau de la portion pouvant être fléchie (16b) et couplées ensemble en série ;
un anneau d'accouplement (63) prévu sur un côté d'extrémité le plus distal de la portion flexible (16c) et couplé à une pièce d'extrémité proximale (50B), qui est une pièce pouvant fléchie prévue sur un côté d'extrémité le plus proximal parmi les pièces pouvant être fléchies (50) ;
un élément convergent (70) comprenant : des portions de conduite de ramification (71a ; 71b) constituées d'une extrémité proximale de l'élément convergent présentant une ramification séparée, et communiquant respectivement avec le tube d'alimentation en gaz (73) et le tube d'alimentation en liquide (74), et une portion de conduite convergente (72) prévue en continu avec les portions de conduite de ramification ;
un tube d'alimentation en gaz/liquide (28) qui permet à la buse à jet de fluide (27) et à la portion de conduite convergente de communiquer l'une avec l'autre, et
un élément intégré (29) à introduire à travers la portion pouvant être fléchie (16b) et la portion flexible (16c) conjointement à l'élément convergent ;
dans laquelle la pièce d'extrémité proximale présente à l'intérieur un espace où entre la portion de conduite convergente ou le tube d'alimentation en gaz/liquide (28) installé sur une surface périphérique extérieure de la portion de conduite convergente,
**caractérisée en ce que** le tube d'alimentation en gaz/liquide présente une longueur telle qu'au moins une portion de la portion de conduite convergente (72) demeure dans l'anneau d'accouplement (63) lorsque l'élément convergent est attiré dans la portion d'extrémité distale par la portion pouvant être fléchie qui est fléchie, et une entrée des portions de conduite de ramification (71a ; 71b) dans la pièce d'extrémité proximale est empêchée en raison de l'aboutement des portions de conduite de ramification (71a ; 71b) contre l'élément intégré (29).

2. Portion d'introduction d'endoscope (16) selon la revendication 1,
dans laquelle une partie de la portion de conduite convergente se trouve dans la portion flexible (16c) dans un état où la portion pouvant être fléchie (16b) est dans un état linéaire.

3. Portion d'introduction d'endoscope (16), comprenant : une portion d'extrémité distale (16a) ; une portion pouvant être fléchie (16b), et une portion flexible (16c), de manière séquentielle à partir d'une extrémité distale à partir de la portion d'introduction d'endoscope (16), la portion d'introduction d'endoscope (16) comprenant en outre :
une buse à jet de fluide (27), prévue au niveau de la portion d'extrémité distale (16a), la buse à jet (27) étant apte à projeter un gaz et un liquide à partir d'elle ;
un tube d'alimentation en gaz (73) et un tube d'alimentation en liquide (74) apte à alimenter le gaz et le liquide respectivement à partir d'un dispositif d'alimentation en gaz/liquide (13) prévu à l'extérieur de la portion d'introduction d'endoscope (16) ;
une pluralité de pièces pouvant être fléchies (50) prévues au niveau de la portion pouvant être fléchie (16b) et couplées ensemble en série ;
un anneau d'accouplement (63) prévu sur un côté d'extrémité le plus distal de la portion flexible (16c) et couplé à une pièce d'extrémité proximale (50B), qui est une pièce pouvant fléchie prévue sur un côté d'extrémité le plus proximal parmi les pièces pouvant être fléchies (50) ;
un élément convergent (70) comprenant : des portions de conduite de ramification (71a ; 71b) constituées d'une extrémité proximale de l'élément convergent présentant une ramification séparée, et communiquant respectivement avec le tube d'alimentation en gaz (73) et le tube d'alimentation en liquide (74), et une portion de conduite convergente (72) prévue en continu avec les portions de conduite de ramification ;
un tube d'alimentation en gaz/liquide (28) qui permet à la buse à jet de fluide (27) et à la portion de conduite convergente de communiquer l'une avec l'autre, et
un fil de manoeuvre pouvant être fléchi (51), apte à manoeuvrer la portion pouvant être fléchie par flexion,
**caractérisée en ce que** le tube d'alimentation en gaz/liquide (28) présente une longueur telle qu'au moins une portion de la portion de conduite convergente (72) demeure dans l'anneau d'accouplement (63) lorsque l'élément convergent est attiré dans la portion d'extrémité distale par la portion pouvant être fléchie qui est fléchie, et par
un élément guide (55) apte à guider le fil de manoeuvre pouvant être fléchi (51), et
une portion de fixation (60) prévue à l'intérieur de la pièce d'extrémité proximale et adaptée à l'élément guide,
dans laquelle l'entrée de la portion de conduite de ramification (71 a ; 71 b) dans la pièce d'extrémité proximale est empêchée en raison de l'aboutement de la portion de conduite de ramification (71a ; 71 b) contre la portion de fixation (60).

4. Portion d'introduction d'endoscope (16) selon la revendication 3,
dans laquelle une partie de la portion de conduite convergente se trouve dans la portion flexible (16c) dans un état où la portion pouvant être fléchie (16b) est dans un état linéaire.

5. Portion d'introduction d'endoscope (16) selon la revendication 3 ou 4,
dans laquelle la portion de fixation est constituée d'une portion fléchie formée par la flexion d'une partie d'une portion cylindrique de la pièce d'extrémité proximale (50B) vers l'intérieur, la portion de fixation présentant une portion saillante (76) formée d'un seul tenant avec la portion fléchie et faisant en sorte de faire saillie vers l'élément convergent, et
dans laquelle la portion de fixation (60) empêche l'entrée de la portion de conduite de ramification (71a ; 71b) dans la pièce d'extrémité proximale en raison de l'aboutement de la portion de conduite de ramification contre la portion saillante (76).

6. Portion d'introduction d'endoscope (16) selon l'une quelconque des revendications 3 à 5, comprenant en outre :
un élément de sollicitation (80) prévu entre l'élément guide (55) et l'élément convergent, et apte à solliciter l'élément convergent vers un côté d'extrémité proximal de la portion flexible pour empêcher l'entrée de la portion de conduite de ramification (71 a ; 71 b) dans la pièce d'extrémité proximale.

7. Portion d'introduction d'endoscope (16) selon l'une quelconque des revendications 1 à 6,
dans laquelle le tube d'alimentation en gaz/liquide (28), le tube d'alimentation en liquide (74), le tube d'alimentation en gaz (73) et l'élément convergent sont formés d'un seul tenant.

8. Endoscope (10) comprenant :
la portion d'introduction d'endoscope (16) selon l'une quelconque des revendications 1 à 7,
la portion de manoeuvre à la main (17) est prévue en continu avec la portion d'introduction d'endoscope (16), et
un élément de manoeuvre de flexion (22,23) apte à réaliser la flexion de la portion pouvant être fléchie (16b).
